Europäisches Patentamt

**European Patent Office**

Office européen des brevets

(19)

(11) Veröffentlichungsnummer : **0 043 825**
**B1**

(12) ## EUROPÄISCHE PATENTSCHRIFT

(45) Veröffentlichungstag der Patentschrift :
21.03.84

(21) Anmeldenummer : 81900218.9

(22) Anmeldetag : 15.01.81

(86) Internationale Anmeldenummer :
PCT/DE 81/00014

(87) Internationale Veröffentlichungsnummer :
WO WO/81019 (23.07.81 Gazettee 81/17)

(51) Int. Cl.³ : **A 61 K   6/08**

(54) VERFAHREN ZUR HERSTELLUNG VON ZAHNERSATZTEILEN DURCH PHOTOPOLYMERISIEREN EINER VERFORMBAREN MASSE.

(30) Priorität : 17.01.80 DE 3001616

(43) Veröffentlichungstag der Anmeldung :
20.01.82 Patentblatt 82/03

(45) Bekanntmachung des Hinweises auf die Patenterteilung : 21.03.84 Patentblatt 84/12

(84) Benannte Vertragsstaaten :
AT CH DE FR GB LI NL SE

(56) Entgegenhaltungen :
EP-A- 0 002 831
DE-A- 2 419 887
DE-B- 1 516 456
FR-A- 2 156 760
US-A- 3 756 827

(73) Patentinhaber : Espe Fabrik pharmazeutischer Präparate GmbH
D-8031 Seefeld / Obb (DE)

(72) Erfinder : JOCHUM, Peter
Pointweg 5
D-8031 Hechendorf (DE)
Erfinder : GASSER, Oswald
Hartstrasse 13
D-8031 Seefeld (DE)

(74) Vertreter : Wuesthoff, Franz, Dr.-Ing. et al
Patentanwälte Wuesthoff -v. Pechmann-Behrens-Goetz Schweigerstrasse 2
D-8000 München 90 (DE)

## Verfahren zur Herstellung von Zahnersatzteilen durch Photopolymerisieren einer verformbaren Masse

Zur Herstellung der verschiedenen Zahnersatzteile, wie Ganzkronen, Verblendung von Metallkronen sowie des sichtbaren Teils von dentalen Brückenkonstruktionen, werden neben den keramischen Werkstoffen in großem Umfang polymerisierbare Massen auf Basis äthylenisch ungesättigter Verbindungen verwendet. Die damit hergestellten Zahnersatzteile aus Kunststoff werden wegen ihres geringeren Preises, ihrer leichteren Verarbeitung und ihrer geringeren Sprödigkeit in vielen Fällen den Keramikmaterialien vorgezogen, zumal die keramischen Zahnersatzteile auch eine wesentlich höhere Härte als die natürliche Zahnsubstanz besitzen und daher bei ihrer Verwendung der jeweilige Gegenbiß verschleißgefährdet ist.

Als Monomergrundlage für die Herstellung der Kunststoffe und Zahnersatzteile werden als äthylenisch ungesättigte Verbindungen meist Methylmethacrylat sowie di- oder polyfunktionelle Acrylsäureester oder Methacrylsäureester verwendet, wobei zur Verringerung des Polymerisationsschrumpfes und zur Erhöhung der mechanischen Festigkeit sowie zum Farbangleich an die natürlichen Zähne, den Monomeren vor der Polymerisation Füllstoffe, Farbstoffe, Fluoreszenzstoffe und Ähnliches zugesetzt werden. Als Füllstoffe eignen sich u. a. bereits fertigpigmentierte Polymethacrylat-Perlen oder andere pulverisierte organische Polymerisate sowie auch anorganische Füllstoffe, insbesondere mikrofeine Füllstoffe, wie z. B. pyrogene Kieselsäure.

Diese Monomermassen müssen zur Herstellung ästhetisch einwandfreier Zahnersatzteile in einer großen Einfärbepalette bereitgestellt werden. Darunter befinden sich auch sehr helle Farben, z. B. für die Massen, die für den Zahnschneidebereich verwendet werden, weshalb die Ausgangsstoffe die Herstellung völlig farbloser Polymerisate ermöglichen müssen mit einer Gewährleistung der Farbstabilität bis zu mehr als einem Jahrzehnt.

Die Polymerisation und Aushärtung der Monomerzubereitungen kann prinzipiell durch den Zusatz der üblichen Radikalbildner als Polymerisationskatalysatoren erfolgen. Wegen der hohen Anforderungen an die Farbstabilität hat sich nur die Polymerisation in der Hitze unter Verwendung von Peroxiden, z. B. Lauroyl- oder Benzoyl-peroxid bewährt. Wegen der begrenzten Haltbarkeit der fertig zubereiteten Mischung aus Monomer, Füll- und Hilfsstoffen sowie den Peroxiden, werden dem Zahntechniker diese Massen meist als als Zwei-Komponenten-Systeme zur Verfügung gestellt. Hierbei besteht eine der Komponenten aus einer peroxidfreien Monomerzubereitung und die andere aus einer monomerfreien Peroxidzubereitung. Unmittelbar vor der Verarbeitung werden dann die beiden Komponenten vermischt.

Diese Zwei-Komponenten-Systeme sind jedoch wegen des erforderlichen Anmischvorgangs in der Handhabung unbequem, wobei auch falsche Dosierung und vor allem eine inhomogene Mischung sowie Lufteinschlüsse in der Masse zu Fehlern führen können, die den Erfolg der Arbeiten gefährden. Außerdem müssen die nicht verwertbaren Reste der jeweils frisch zuzubereitenden Mischung verworfen werden, da sie nicht haltbar sind.

Man hat daher bereits Ein-Komponenten-Systeme entwickelt, bei denen als Radikalbildner ein Photoinitiatorsystem vorhanden ist. Die Aushärtung der polymerisierbaren Masse erfolgt dann durch Lichtbestrahlung. Derartige, bei Dunkellagerung stabile Präparate haben sich als Zahnfüllmassen in den vergangenen Jahren bewährt. Die auf UV-Licht, insbesondere mit Wellenlänge von 320 bis 400 nm, ansprechenden Photoinitiatoren, wie Benzoinalkyläther oder Benzilmonoketale weisen jedoch unzureichende Farbstabilität besonders in den in Kronen- und Brückenmaterialien benötigten, sehr hellen Einfärbungen auf und führen insbesondere bei den teilweise benötigten dunkleren Einfärbungen der Monomerzubereitungen nur zu unzureichenden Härtungen im Innern der daraus hergestellten Formkörper. Überdies erfordert die Bestrahlung mit UV-Licht technisch aufwendige und daher teure Lampen, die nur eine relativ geringe Lebensdauer aufweisen.

In der DE-OS 29 14 537 sind derartige Mischungen für die Herstellung von Zahnkronen beschrieben, wobei als Photoinitiatoren Benzil und Benzoinalkyläther verwendet werden. Zur Erzielung einer besseren Aushärtung, insbesondere in der Tiefe, werden den polymerisierbaren Massen noch zusätzlich Peroxide beigefügt. Diese Maßnahme beeinträchtigt jedoch wieder entscheidend die Lagerfähigkeit dieser Zubereitung. Bessere Aushärttiefen kann man mit den auf den kurzwelligen Anteil des sichtbaren Lichts bei Wellenlängen von ca. 400-600 nm ansprechenden 1,2-Diketonverbindungen als Photoinitiatoren erreichen, besonders wenn sie zusammen mit Aminen verwendet werden, wie z. B. in der DE-OS 22 51 048 offenbart worden ist. Die meist deutlich gelbe Eigenfarbe der 1,2-Diketoninitiatoren und die den Ansprüchen eines daraus hergestellten Zahnersatzes meist nicht genügende Farbstabilität begrenzt die Anwendung dieser Photosensibilisatoren zur Formulierung eines Ein-Komponenten-Materials, das zur Herstellung von Zahnersatzteilen, wie Kronen und Brücken geeignet ist.

Aufgabe der Erfindung ist daher, ein Verfahren zur Herstellung von Kronen und Brückenkonstruktionen oder dergleichen Zahnersatzteilen aus Kunststoffen bereitzustellen, das allen Anforderungen, insbesondere hinsichtlich der Farbstabilität und Einfärbemöglichkeit der Zahnersatzteile gerecht wird und die Nachteile und Fehlermöglichkeiten der bekannten Zwei-Komponenten-Systeme ausschließt.

Diese Aufgabe wird nun dadurch gelöst, daß man bei der Herstellung von Zahnersatzteilen durch Photopolymerisieren einer verformbaren Masse, die (Meth)Acrylsäureester als polymerisierbare Monomerverbindung und als Photoinitiator 1,2-Diketone, sowie gegebenenfalls Füllstoffe, Farbstoffe und Inhibitoren enthält, erfindungsgemäß die Masse, die als 1,2-Diketon eine Bicyclo-[2.2.1]-heptan dion-2,3-Verbindung enthält, in mehreren Schichten aufbaut, die jeweils durch Bestrahlung polymerisiert werden, und dann das erhaltene Zahnersatzteil so lange weiter bestrahlt, bis der Photoinitiator ausgebleicht ist. Es wurde nämlich erkannt, daß die gelbe Eigenfarbe dieser wirksamen Photoinitiatoren durch eine intensive Beleuchtung, die der eigentlichen Polymerisationsbelichtung nachgeschaltet wird, völlig ausgebleicht werden kann, so daß hinsichtlich Farbgebung und Farbstabilität dann einwandfreie Polymerisatformkörper entstehen. Dieser spezielle « Ausbleicheffekt » bei den erfindungsgemäß zu verwendenden Initiatoren war dem Stand der Technik nicht zu entnehmen.

In der FR-A-2 156 760 (entsprechend DE-A-22 51 048) sind zwar bereits 1,2-Diketonverbindungen als Photoinitiatoren aufgeführt, unter denen auch ein Vertreter der Bicyclo-[2.2.1]-heptan-dion-2,3-Verbindungen, nämlich das Campherchinon, genannt ist. Die polymerisierbare Monomergrundlage ist jedoch kein (Meth)Acrylsäureester, sondern ein Gemisch aus Styrol und ungesättigtem Polyesterharz. Außerdem ist keine Ausbleichstufe nach der Primärbelichtung nachgeschaltet, so daß gelblich gefärbte Produkte entstehen. Auch in der DE-A-2 419 887 wird Campherchinon als Photoinitiator eingesetzt, der dort als Initiator für die Polymerisation bestimmter Vorpolymerisate aus Urethanverbindungen und reaktionsfähigen äthylenisch ungesättigten Monomeren dient. Auch ist keine Ausbleichstufe vorhanden, denn es wird mit einer relativ schwachen Lichtquelle nur für kurze Zeit belichtet. Außerdem unterscheidet sich das erfindungsgemäße Verfahren von diesem Stand der Technik durch den Aufbau des Zahnersatzteils aus mehreren Schichten, die nacheinander polymerisiert und dann zur Ausbleichung weiter bestrahlt werden.

U. Meinwalt und H. O. Klingele (J. Amer. Chem. Soc. 1966, S. 2071-3) haben berichtet, daß bei der Belichtung von Lösungen des Campherchinons in unterschiedlichen Lösungsmitteln in der Gegenwart von Sauerstoff eine Vielfalt von Oxidations- und Reduktionsprodukten gebildet werden, wie z. B. Campher-säure-anhydrid, Endo-3-hydroxycampher, Endo-3-hydroxyepicampher oder Camphonolacton.

Aus diesen Untersuchungen konnte jedoch keineswegs erwartet werden, daß die weitere Belichtung in der Kunststoffmatrix glatt völlig farblose Produkte liefern würde.

Die Festigkeitseigenschaften des Kunststoffmaterials werden hierbei nicht vermindert, denn nur die Initiatorverbindungen werden bei der Nachbelichtung abgebaut. Da die Ausbleichung dieser bestimmten Bicyclodiketone auch in Gegenwart von lichtstabilen Pigmenten eintritt, kann man durch Anwendung des erfindungsgemäßen Verfahrens wunschgemäß eingefärbte Zahnersatzteile herstellen, die ihre Farbtönung auch über Jahre hinaus nicht verändern. Für opake Weißpigmentierung der Ersatzteile im Frontbereich des Gebisses hat sich hierbei das Calcium-fluorid nicht nur als Füllstoff, sondern auch als besonders geeignetes Weißpigment erwiesen, weil es besonders durchlässig ist für das zur Härtung der photopolymerisierbaren Massen geeignete Licht, mittels dessen dann auch nach der Härtung die Ausbleichung der vorhandenen Gelbtönung bei den erfindungsgemäß verwendeten Bicycloketonen ohne Schwierigkeit durchgeführt werden kann.

Auch beim erfindungsgemäßen Herstellungsverfahren für die farbstabilen Zahnersatzteile werden Ester der Acryl- und Methacrylsäure als polymerisierbare Bestandteile der photopolymerisierbaren Massen eingesetzt. Vorzugsweise werden die (Meth-) acrylester von di- oder polyfunktionellen Alkoholen, wie sie z. B. in der DE-OS 28 16 823 oder der DE-PS 19 21 869 beschrieben sind, allein oder im Gemisch mit anderen mehrfunktionellen oder monofunktionellen (Meth-)acrylaten verwendet. Hierbei können neben den üblichen Stabilisatoren, Farb- und Fluoreszenzstoffen, die bekannten organischen oder anorganischen Füllstoffe oder deren Gemische eingesetzt werden. Als organische Füllstoffe eignen sich bevorzugt vorpigmentierte Polymethacrylat-Perlen, während als anorganische Füllstoffe Quarz- oder Silikatgläser in feinvermahlener Form oder mineralische Füllstoffe, wie z. B. Calciumfluorid, brauchbar sind. Besonders gute Ergebnisse werden durch die alleinige oder Mitverwendung von mikrofeinen Füllstoffen, wie z. B. pyrogener Kieselsäure, erhalten. Die genannten Bestandteile werden auch bei dem erfindungsgemäßen Verfahren jeweils in den üblichen Mengen eingesetzt.

Die verwendeten Füllstoffe können in bekannter Weise auch silanisiert sein, um die Bindung mit dem Polymer zu verbessern. Als Silan wird z. B. Trimethoxy-(3-methacryloxypropyl)-silan verwendet.

Zur Vermeidung vorzeitiger Polymerisation können den Massen weiterhin Inhibitoren wie z. B. p-Methoxyphenol in den üblichen Konzentrationen zugesetzt werden.

Die Farbstabilität der Polymerisate unter Sonnenlichteinwirkung kann durch den Zusatz bekannter UV-Stabilisatoren verbessert werden. Diese Stabilisatoren sollen oberhalb einer Wellenlänge von ca. 350 nm nicht mehr in nennenswertem Maße absorbieren, um den Lichthärtungsvorgang nicht zu beeinträchtigen ; geeignet sind z. B. 2-Hydroxy-4-methoxy-benzophenon oder Ethyl-2-cyano-3,3-diphenylacrylat.

Die erfindungsgemäß zu verwendenden Bicyclo-[2.2.1]-heptandion-2,3-initiatoren werden in Mengen von 0,01 Gew.% bis 1 Gew.%, vorzugsweise in einer Konzentration von 0,05 Gew.% bis 0,5 Gew.%, insbesondere 0,1-0,3 % den Dentalmassen zugesetzt. Beispiele für derartige Bicyclo-[2.2.1]-heptandion-2,3-Verbindungen sind das Norcampherchinon (Bicyclo-[2.2.1]-heptan-dion-2,3), das Campherchinon (1,7,7-Trimethyl-bicyclo-[2.2.1]-heptan-dion-2,3) und das Tricyclo-[5.2.1.0$^{2,6}$]-decan-dion-8,9.

Als besonders geeigneter Initiator aus der Reihe dieser Bicycloheptandion-Verbindungen hat sich beim erfindungsgemäßen Verfahren das Campherchinon bewährt, das in bevorzugter Weise zusammen mit den aktivierenden Aminverbindungen eine schnelle Polymerisation bei der Belichtung der Monomeren in den Dentalmassen bewirkt und dann nach der Polymerisation relativ schnell durch weitere Belichtung ausgebleicht werden kann. Als Aminkomponente sind vor allem tertiäre Amine geeignet, die vorzugsweise substituierte Kohlenwasserstoffreste tragen. Besonders bevorzugt sind tertiäre Amine mit drei hydroxysubstituierten Kohlenwasserstoffgruppen, wie Triäthanolamin oder seine Derivate. Die Amine werden in Konzentrationen von 0,1 Gew.% bis 10 Gew.%, vorzugsweise 0,5 Gew.% bis 5 Gew.%, der Masse zugefügt.

Beim Zubereiten der Monomermasse werden das Diketon und gegebenenfalls das Amin im Monomeren gelöst und mit den weiteren Komponenten in einfacher Weise vermischt, z. B. durch Rühren oder Kneten, und zu einer streichfähigen Masse verarbeitet. Dabei ist darauf zu achten, daß während der Herstellung und beim Lagern ein Lichtzutritt zu der photosensiblen Dentalmasse verhindert wird. Durch Rühren unter Evakuieren oder Bearbeitung mit einem Walsenstuhl oder ähnliche Maßnahmen wird ein homogenes, blasenfreies Material hergestellt und in lichtundurchlässige Behälter abgefüllt.

Die Herstellung des Zahnersatzteils geschieht vorzugsweise ähnlich wie beim Verfahren gemäß DE-AS 15 16 456, bei dem auf eine Unterlage die Halbflüssige oder plastische Monomermasse schichtweise aufgetragen wird, wobei man jede Schicht durch eine Wärmehandlung aushärtet, bevor die nächste Schicht aufgetragen wird. Beim erfindungsgemäßen Verfahren wird dabei auf das Brückengerüst oder einen Modellzahnstumpf eine Schicht der Monomermasse gewünschter Farbgebung mit einem geeigneten Instrument, z. B. einem Pinsel oder einem Spatel, aufgetragen.

Anschließend wird mit einer intensiven Lichtquelle belichtet, worauf eine neue Materialschicht aufgetragen und belichtet wird. Durch Auftragen der erforderlichen Schichten kann so ein Zahnersatzteil modelliert werden, das hohen kosmetischen Anforderungen genügt. Mit einer intensiven, sichtbares Licht abgebenden Lichtquelle (z. B. 75-Watt-Halogen-Projektorlampe) beträgt hierbei die Belichtungszeit für jede Schicht zwischen 1 Sekunde und 20 Sekunden, in den meisten Fällen sind ca. 5 Sekunden ausreichend. Das fertig modellierte Zahnersatzteil wird dann gegebenenfalls unter Kühlung der weiteren Bestrahlung ausgesetzt, vorzugsweise dem Licht einer Lampe, die eine hohe Leistung im Wellenbereich zwischen 400 und 500 nm abgibt. Die Belichtung wird so lange fortgesetzt, bis der gelbe Farbton der Bicycloheptadion-Verbindung restlos verschwunden ist. Die erforderliche Belichtungszeit beträgt hierbei je nach Strahlungsintensität zwischen wenigen Minuten und einigen Stunden. Um eine Oberflächenschädigung der Polymerisate durch die Inhibitorwirkung des Luftsauerstoffs auszuschließen, kann die Nachbelichtung auch unter Vakuum erfolgen ; vorzugsweise wird die Nachbelichtung bei einem Druck von < 10 mbar ausgeführt. Die derartig aufgebauten Polymerisatgegenstände zeichnen sich dann durch sehr hohe Farbstabilität aus. Auf diese Weise lassen sich Jacket-Kronen, Brückenverblendungen, Kunststoffbrücken, Kunststoffinlays, orthodontische Präparate und provisorische Kronen und Brücken herstellen.

Die Ausbleichfähigkeit durch die nachbehandlung ist eine Besonderheit der beim erfindungsgemäßen Verfahren verwendeten Bicyclo-[2.2.1]-heptan-dion-2,3-Verbindungen aus der Reihe der 1,2-Diketon-Initiatoren für die Photopolymerisation. Dies geht deutlich aus den Ergebnissen der nachstehend beschriebenen Versuchsreihe hervor.

Es wurden durch Vermischen von jeweils 35 Gew.% Hexandioldiacrylat, 35 Gew.% Bis-hydroxymethyl-tri-cyclo-[5.2.1.0$^{2,6}$]-decan-diacrylat und 30 Gew.% silanisierter, pyrogener Kieselsäure sowie 0,2 Gew.% verschiedener 1,2-Diketon-Photoinitiatoren und 1,5 Gew.% Triäthanolamin verformbare plastische Zubereitungen hergestellt. Durch Einfüllen in eine entsprechende Kunststofform und 40 sekundenlanges Belichten mit dem sichtbaren Lichtanteil einer 75-Watt-Halogen-Projektorlampe wurden zylindrische Probekörper von 15 mm Durchmesser und 2 mm Dicke hergestellt. Anschließend wurden die Probekörper unter Luftkühlung 1 Stunde dem Licht einer Lampe, die hohe Leistung im Wellenlängenbereich zwischen 400 und 500 nm abgibt, ausgesetzt.

Der Farbeindruck wurde visuell beurteilt und ist in der nachfolgenden Tabelle wiedergegeben :

| Verwendetes 1,2-Diketon | Endfarbe des Probekörpers | Bemerkung |
|---|---|---|
| a) Campherchinon | Völlig farblos | Erfindungsgemäß |
| b) Phenanthrenchinon | Kanariengelb | |
| c) Benzil | Stark gelb | |
| d) α-Naphtil | Stark rot | |
| e) p,p'-Dichlorbenzil | Gelb | |
| f) Acenaphtenchinon | Stark rot | |
| g) p,p'-Dimethoxybenzil | Gelb | |
| h) Diacetyl | Leicht gelb | Flüchtig, sehr unangenehmer Geruch |
| i) 3,4-Hexandion | Leicht gelb | Sehr unangenehmer Geruch |
| j) 1,2-Cyclohexandion | Leicht gelb | Keine Polymerisation |
| k) Furil | Stark gelb | Keine Polymerisation |
| l) 2-Naphtil | Stark gelb | Keine Polymerisation |
| m) p-Nitrobenzil | Stark gelb | Keine Polymerisation |
| n) 4,4-Dimethylbenzil | Gelb | Keine Polymerisation |

**0 043 825**

Es ist ersichtlich, daß von der Vielzahl der in der DE-OS 22 51 048 genannten 1,2-Diketone nur das Campherchinon zur Formulierung einer photohärtbaren Masse brauchbar ist, die farblose Zahnersatzteile liefern kann. Die allen gemeinsame mehr oder weniger starke gelbe Eigenfärbung der 1,2-Diketone läßt sich nämlich nur im Falle des Campherchinons durch intensive Belichtung der auspolymerisierten Masse völlig ausbleichen. Bei den anderen 1,2-Diketonen tritt dieser « Ausbleicheffekt » nicht ein ; sie haben Verfärbungen gezeigt, die von leicht gelblich nach stark rot gehen ; einige Vertreter der 1,2-Diketone sind durch ihre hohe Flüchtigkeit und ihren sehr unangenehmen Eigengeruch zusätzlich als ungeeignet zu bezeichnen.

Die Verwendung des Photoinitiatorsystems aus den bestimmten Bicycloheptandion-Verbindungen und vorzugsweise einem Amin ermöglicht es, den Zahntechnikern dunkellagerbeständige Ein-Komponenten-Systeme zur Verfügung zu stellen, die außer der Farbstabilität noch weitere Vorteile haben. Beim üblichen Heißpolymerisationsverfahren entstehen beim Aufbringen der Kunststoffmasse auf eine Metallunterlage (z. B. aus Gold oder Platinlegierung) durch das Erhitzen während der Polymerisation und das anschließende Abkühlen wegen der unterschiedlichen thermischen Ausdehnungskoeffizienten von Metall und Kunststoff Spannungen und Spalte an der Verbindungsstelle, die die Festigkeit der Zahnersatzteile vermindern. Da die Photopolymerisation mit vergleichsweise geringer Temperaturerhöhung verläuft, treten diese Erscheinungen kaum auf.

Außerdem werden Fehldosierungen, inhomogene Mischungen, sowie Lufteinschlüsse und ähnliche Mischfehler vermieden. Auch müssen keine Restmengen verworfen werden, so daß das erfindungsgemäße Material in seiner Verwendung sehr ökonomisch ist.

Beispiel 1

Eine Lösung wird bereitet aus

20 g Hexandioldiacrylat
20 g Bis-hydroxymethyl-tricyclo [5.2.1.0$^{2,6}$]-decan-diacrylat
und
60 mg 1,7,7-Trimethyl-bicyclo [2.2.1]-heptandion-2,3 (Campherchinon).

Eine Pulvermischung wird hergestellt aus

14 g zahnähnlich eingefärbter, silanisierter, pyrogener Kieselsäure
und
4 g Calciumfluorid.

Die Flüssigkeit wird in einem Laborkneter vorgelegt und das Pulvergemisch langsam zugegeben. Nach einer Knetzeit von ca. 1 h wird die erhaltene Masse auf einem Walzenstuhl weiter homogenisiert und anschließend unter Vakuum erneut geknetet bis ein blasenfreies, verstreichbares Produkt erhalten wird, das eine gewisse Thixotropie aufweist.

Wenn die Masse hell eingefärbt ist, wie man sie für den Schneidebereich von Zahnersatzteilen benötigt (entsprechend der Farbe S11 des « Biodent »-Farbringes der Firma De Trey), härtet sie nach 20 sekundenlanger Bestrahlung mit dem Licht einer handelsüblichen, sichtbares Licht emittierenden Lampe für den dentalen Gebrauch (Elipar-Gerät, Firma ESPE) in einer Schichtstärke von 8 mm aus.

Die Druckfestigkeit des Polymerisats wird an Prüfkörpern von 2 × 2 × 4 mm gemessen, die in geeigneten Formen durch Primärbelichtung (20 sec.) mit dem oben genannten dentalen Belichtungsgerät hergestellt werden. Die zunächst deutlich gelb gefärbten Körper werden 60 min. dem Licht einer Lichtquelle ausgesetzt, die mit hoher Intensität den Wellenlängenbereich zwischen 400 und 500 nm abstrahlt. Die nun erhaltenen, den reinen Farbton der gewählten Einfärbung aufweisenden Formkörper haben nach 24-stündiger Lagerung unter Wasser bei 36 °C eine Druckfestigkeit von 410 MPa.

Beispiel 2

Entsprechend Beispiel 1 wird ein Kronen- und Brückenmaterial hergestellt, das als Photoinitiator statt Campherchinon Tricyclo [5.2.1.0$^{2,6}$]-decan-dion-8,9 enthält, hergestellt in bekannter Weise durch Oxidation des Tricyclo-[5.2.1.0$^{2,6}$]-decan-8-ons mit Selendioxid, und in heller Zahnschneide-Farbe (entsprechend der Farbe S11 des « Biodent »-Farbringes der Fa. De Trey) eingefärbt ist. Das resultierende Material härtet bei Belichtung wie in Beispiel 1 beschrieben in einer Schichtdicke von 7 mm aus (Elipar-Lampe, Fa. ESPE). In einer geeigneten Kunststoff-form wird ein zylindrischer Prüfkörper von 15 mm Durchmesser und 1,5 mm Dicke durch eine Primärbelichtung von 40 sec. hergestellt.

Nach einer Sekundärbelichtungszeit von 1 Stunde unter dem Licht einer Lampe, die eine hohe Intensität im Bereich zwischen 400 und 500 nm emittiert, weist der zunächst gelb gefärbte Prüfkörper die reine Farbe der gewählten Einfärbung auf.

5

## Beispiel 3

Entsprechend Beispiel 1 wird ein Kronen- und Brückenmaterial hergestellt, das als Photoinitiator statt Campherchinon Bicyclo-[2.2.1]-heptan-dion-2,3 (Norcampherchinon) enthält und in heller Zahnschneide-Farbe (entsprechend der Farbe S11 des « Biodent »-Farbringes der Firma De Trey) eingefärbt ist.

Ein wie in Beispiel 2 hergestellter Prüfkörper ist zunächst gelb gefärbt und weist nach der entsprechenden Sekundärbelichtung (1 Stunde) den reinen Farbton der gewählten Einfärbung auf.

## Beispiel 4

In gleicher Weise wie in Beispiel 1 wird ein Kronen- und Brückenmaterial hergestellt, bei dessen Zubereitung der Lösung 360 mg Triethanolamin-tris-phenylurethan als Aktivator zugesetzt werden. Die Masse wird in einem dunklen Braun eingefärbt, das zur Herstellung der Zahnhälse bei Zahnersatzteilen benötigt wird (entsprechend der Farbe H32 des « Biodent »-Farbringes der Firma De Trey).

Nach 20-sekündiger Belichtung härtet das Material in einer Schichtstärke von 4 mm aus. Seine Druckfestigkeit beträgt 430 MPa (gemessen wie in Beispiel 1).

## Beispiel 5

Herstellung einer Jacket-Krone

Entsprechend Beispiel 4 werden materialien in den gewünschten Einfärbungen für Zahnhals, Dentin und Zahnschneide hergestellt.

Auf ein isoliertes Zahnstumpfmodell werden mit einem Pinsel oder einem Spatel Schichten in der Farbe und Stärke aufgetragen, die zur Angleichung an das Aussehen des natürlichen Zahnes erforderlich sind.

Nach dem Auftragen jeder Schicht wird 10 sec. mit einer handelsüblichen Dentallampe hoher Leistung im Wellenlängenbereich 400-500 nm (Elipar-Gerät, Firma ESPE) belichtet und die Masse so zur Polymerisation gebracht. Durch 1-stündige Sekundärbelichtung in einem Vakuum von 5 mbar mittels einer luftgekühlt Lampe, die hohe Leistung im Wellenlängenbereich zwischen 400 und 500 nm aufweist, wird der gelbe Farbton des Campherchinons ausgebleicht. Um Schattenstellen zu vermeiden, muß die Position der Krone gegebenenfalls so verändert werden, daß die gesamte Oberfläche der Krone dem Bestrahlungslicht genügend ausgesetzt ist. Man erhält auf diese Weise eine abrasionsfeste Krone, die dem natürlichen Zahn gleicht und von hoher Farbbeständigkeit ist.

## Beispiel 6

Verblendung einer Edelmetallbrücke

Die nach Beispiel 4 gefertigten Materialien werden in den gewünschten Farbgebungen bereitgestellt.

Auf das Brückengerüst, das mit den üblichen Retentionen versehen und mit Opaker beschichtet ist, werden schichtweise die erforderlichen Farben aufgetragen. Jede Schicht wird, wie in Beispiel 5 beschrieben, durch Belichtung polymerisiert. Auch das hinter den Metallretentionen befindliche Material härtet dabei aus.

Anschließend wird wieder analog Beispiel 5 durch Nachbelichtung ausgebleicht, gegebenenfalls unter Positions-änderung der Brücke, um alle Partien dem Licht auszusetzen.

Die so erhaltenen Verblendungen zeichnen sich durch ihre Farb- und Abrasionsbeständigkeit aus. Außerdem ist ihr Erscheinungsbild kosmetisch völlig einwandfrei.

## Ansprüche

1. Verfahren zur Herstellung von Zahnersatzteilen durch Photopolymerisieren einer verformbaren Masse, die Acryl- und/oder Methacrylsäureester als polymerisierbare Monomerverbindung und als Photoinitiator 1,2-Diketone, sowie gegebenenfalls Füllstoffe, Farbstoffe und Inhibitoren enthält, dadurch gekennzeichnet, daß man die Masse, die als 1,2-Diketon eine Bicyclo [2.2.1]-heptan-dion-2,3-Verbindung enthält, in mehreren Schichten aufbaut, die jeweils durch Bestrahlung polymerisiert werden, und dann das erhaltene Zahnersatzteil so lange weiter bestrahlt, bis der Photoinitiator ausgebleicht ist.

2. Verfahren nach Anspruch 1, dadurch gekennzeichnet, daß die Masse 0,01-1 Gew.%, vorzugsweise 0,05-0,5 Gew.% Bicyclo [2.2.1]-heptan-dion-2,3-Verbindung enthält.

3. Verfahren nach Anspruch 1 und 2, dadurch gekennzeichnet, daß die Masse ein Amin, vorzugsweise ein tertiäres Amin mit 3 substituierten Kohlenwasserstoffresten am Stickstoff, als Aktivator enthält.

4. Verfahren nach Anspruch 1-3, dadurch gekennzeichnet, daß man das Ausbleichen mit Strahlung im Wellenlängenbereich zwischen 400-500 nm durchführt.

5. Verfahren nach Anspruch 4, dadurch gekennzeichnet, daß man als Bicyclo-[2.2.1]-heptan-dion-2,3-Verbindung das Campherchinon verwendet.

6. Verfahren nach Anspruch 1-5, dadurch gekennzeichnet, daß man als Füllstoff pyrogene Kieselsäure verwendet.

### Claims

1. A process for the production of dental prosthesis parts by photopolymerizing a deformable composition containing (meth)acrylic acid esters as polymerizable monomer and as photoinitiator 1,2-diketones, as well as optionally fillers, dyes and inhibitors, characterized in that the composition which contains as 1,2-diketone a bicyclo [2.2.1]-heptane-dione-2,3 is constructed in a plurality of layers each of which is polymerized by radiation, and that then the dental prosthesis part obtained is further radiated until the photoinitiator has faded.

2. The process as claimed in claim 1, characterized in that the composition contains 0.01 to 1 weight percent, preferably 0.05 to 0.5 weight percent of bicyclo [2.2.1]-heptane-dione-2,3.

3. The process as claimed in claims 1 and 2, characterized in that the composition contains an amine, preferably a tertiary amine with 3 substituted hydrocarbon groups at the nitrogen, as activator.

4. The process as claimed in claims 1 to 3, characterized in that the fading is performed by radiation in the wave length range of between 400 and 500 nm.

5. The process as claimed in claim 4, characterized in that as bicyclo-[2.2.1]-heptane-dione-2,3 the campherchinone is used.

6. The process as claimed in claims 1 to 5, characterized in that pyrogeneous silicic acid is used as filler.

### Revendications

1. Procédé de préparation de pièces de prothèse dentaire par photopolymérisation d'une masse déformable, qui contient un ester d'acide acrylique et/ou méthacrylique comme composé monomère polymérisable, et comme photoinducteur de la 1,2-dicétone, ainsi qu'éventuellement des produits de remplissage, colorants et inhibiteurs, caractérisé en ce qu'on construit la masse, qui contient comme 1,2-dicétone un composé de bicyclo [2.2.1]-heptane-dione-2,3, en plusieurs couches, que l'on polymérise à chaque fois par rayonnement, et en ce qu'on irradie alors plus avant la pièce de prothèse dentaire obtenue jusqu'à ce que le photoinducteur soit décoloré.

2. Procédé selon la revendication 1, caractérisé en ce que la masse contient de 0,01 à 1 % en poids, de préférence de 0,05 à 0,5 % en poids de composé de bicyclo [2.2.1]-heptanedione-2,3.

3. Procédé selon l'une des revendications 1 ou 2, caractérisé en ce que la masse contient comme activeur une amine, de préférence une amine tertiaire avec trois radicaux hydrocarbonés substitués sur l'azote.

4. Procédé selon l'une des revendications 1 à 3, caractérisé en ce qu'on conduit la décoloration avec un rayonnement dans un intervalle de longueur d'onde compris entre 400 et 500 nm.

5. Procédé selon la revendication 4, caractérisé en ce qu'on utilise comme composé de bicyclo-[2.2.1]-heptanedione-2,3 la camphoquinone.

6. Procédé selon l'une des revendications 1 à 5, caractérisé en ce qu'on utilise comme produit de remplissage de l'acide silicique pyrogène.